# EUROPEAN PATENT APPLICATION

(11) **EP 1 602 339 A1**
(43) Date of publication of application: **07.12.2005**
(21) Application number: 05251911.3
(22) Date of filing: 29.03.2005
(51) Int. Cl.: A61B 18/14

(54) **Devices, systems, and methods for treating atrial fibrillation**

(30) Priority: 01.04.2004 US 815477
(71) Applicant: A M Discovery, Inc., La Jolla, CA 92037 (US)
(72) Inventor: Macoviak, John A., La Jolla, California 92037 (US); Rahdert, David A., San Francisco, CA 94116 (US)
(74) Representative: Dee, Ian Mark

(57) **Abstract**

Devices, systems and methods establish scaffold-like structures within the heart or a body cavity. The scaffold-like structures establish a stable platform that makes it possible to accomplish a desired therapeutic and/or diagnostic objective in a targeted fashion in one or more tissue regions. The desired therapeutic and/or diagnostic objectives can include, e.g., (i) the delivery of various chemical, drug, and/or biological agents (in liquid, gel, or wafer form) into contact on or in the tissue regions, to accomplish, e.g., ablation or other therapeutic objectives in a controlled, precise fashion; and/or (ii) the delivery of mechanical, heat, cooling, or electrical energy to the tissue region - e.g., to promote the intake by tissue of a chemical, drug, or biological agent, or to pace heart tissue, or to form temporary or permanent conduction blocks, or to otherwise achieve localized effects in targeted tissue regions such as tissue shrinkage, or combinations thereof; and/or (iii) the positioning of physiologic sensors and/or monitors to analyze heart function for diagnostic purposes, e.g., to locate the origin of ectopic pacemakers or other regions where therapeutic intervention may be indicated.

## Description

### Related Applications

This application claims the benefit of copending United States Patent Application Serial No. 10/491,461 , filed April 1, 2004 and entitled "Devices for Treating Atrial Fibrillation," which is a continuation of PCT Patent Application No. WO 03/028802 (PCT/US02/31374), filed October 1, 2002 and entitled "Devices for Treating Atrial Fibrillation," which claims the benefit of United States Provisional Application Serial No. 60/326,590, filed October 1, 2001, which are incorporated herein by reference.

### Field of the Invention

The invention is directed to devices, systems, and methods for improving the function of the heart. In a more particular sense, the invention also relates to devices, systems, and methods for treating atrial fibrillation.

### Background of the Invention

To function properly as a pump, the heart must contract in a rhythmic pattern. Heart rhythm is normally established at a single point called the sinoatrial node, or SA node, located in the right atrium of the heart, near the opening of the superior vena cava. The SA node generates electrical impulses which spread throughout the heart and result in a rhythmic contraction of the heart, termed a sinus rhythm. Thus, the SA node functions as a pacemaker for the heart.

Other regions of the heart can potentially produce electrical impulses. A pacemaker other than the SA node is referred to as an ectopic pacemaker. Electrical signals from an ectopic pacemaker can disrupt a rhythmically contracting heart, resulting in an arrhythmia, characterized by a chaotic, disorganized heart rhythm. Fibrillation of the atria results in loss of atrial contraction and rapid impulses being sent to the ventricles causing high and irregular heart rates. Atrial fibrillation (AF) is clinically related to several conditions, including anxiety, increased risk of stroke due to blood stasis in the atrium that then clots and forms emboli, reduced exercise tolerance, cardiomyopathy, congestive heart failure and decreased survival. Patients who experience AF are, generally, acutely aware of the symptoms.

Current curative AF therapies are based upon a procedure that has become known as the Cox Maze procedure. The Cox Maze procedure is an open-heart, surgical procedure that requires the patient to be placed on cardiopulmonary bypass equipment. The procedure requires six hours and the patient to be under general anesthesia. In this procedure, access to the heart is gained by way of a median sternotomy, which is a surgical split of the breast bone. The left atrium is surgically incised along predetermined lines known to be effective in blocking the transmission of electrical signals from an ectopic pacemaker that triggers AF. The incision lines create blocks that prevent conduction of unwanted electrical signals throughout the heart and permit a normal pattern of depolarization of the atria and ventricles beginning in the SA node and traveling to the AV or atrioventricular node.

There is also a focal ablation therapy that is endovascular in its approach to the left atrium, usually going through a puncture in the atrial septum after initial access in a peripheral vein like the femoral vein. This form of treatment has had limited success in applying heat and cold tissue ablation techniques to sites inside a pulmonary vein. This focal ablation technique is useful for a small group of patients who have paroxysmal atrial fibrillation and in whom one or two initiation culprit sites of atrial activation can be mapped using complicated electrophysiology equipment that can entail up to eight hours of mapping. Focal ablation has had no significant success in ablating chronic or persistent atrial fibrillation.

All endovascular technologies to date have been unable to replicate ablation of the major tissue tracts that are necessary to cure chronic atrial fibrillation, as can be done successfully with the Cox-Maze open heart surgical approach.

A therapy using thoracoscopic or small incision in the thorax, not requiring cardiopulmonary bypass, is being used to access the epicardial surface of the left atrium. This therapy is under study to apply primarily energy based ablation techniques to encircle the four pulmonary veins as a group. Its safety and efficacy are unknown.

There is a need for new therapeutic tissue conduction ablation agents and for less invasive methods and devices for treating AF, to thereby improve heart function and improve patient safety.

### Summary of the Invention

The invention provides devices, systems and methods that establish scaffold structure within the heart or a body cavity.

According to one aspect of the invention, the scaffold structure is sized and configured to rest within a left atrium between a septum wall and an opposite lateral wall.

In one embodiment, the scaffold structure supports a component sized and configured to rest adjacent to tissue on a posterior wall essentially surrounding a pulmonary vein region. The scaffold structure is free of a component that contacts tissue along an anterior wall of the left atrium.

In one embodiment, the scaffold structure supports a first component sized and configured to rest adjacent to tissue on a posterior wall essentially surrounding a pulmonary vein region. The scaffold structure also supports a second component configured to rest adjacent to tissue on the posterior wall and overlay a portion of a coronary sinus and a portion of a posterior mitral valve annulus within the left atrium.

In either embodiment, the scaffold structure establishes a stable platform that makes it possible to accomplish a desired therapeutic and/or diagnostic objective in a targeted fashion in one or more tissue regions. The desired therapeutic and/or diagnostic objectives can include, e.g., (i) the delivery of various chemical, drug, and/or biological agents (in liquid, gel, or wafer form) into contact on or in the tissue regions, to accomplish, e.g., ablation or other therapeutic objectives in a controlled, precise fashion; and/or (ii) the delivery of mechanical, heat, cooling, or electrical energy to the tissue region - e.g., to promote the intake by tissue of a chemical, drug, or biological agent, or to pace heart tissue, or to form temporary or permanent conduction blocks, or to otherwise achieve localized effects in targeted tissue regions such as tissue shrinkage, or combinations thereof; and/or (iii) the positioning of physiologic sensors and/or monitors to analyze heart function for diagnostic purposes, e.g. to establish uniform contact between the sensors/monitors and cardiac tissue intended to be analyzed or treated.

According to another aspect of the invention, a heart treatment device comprises a scaffold structure sized and configured to rest adjacent to heart tissue essentially surrounding a pulmonary vein region. The scaffold structure supports an ablation agent delivery site and at least one catalytic energy source to affect intake of ablation agent by tissue. The scaffold structure can be sized and configured to contact epicardial tissue. Alternatively, the scaffold structure can be sized and configured to contact endocardial tissue.

The scaffold structures that embody features of the invention can be made of biocompatible metallic or polymeric materials, or combinations thereof. The scaffold structures can be deployed using conventional open heart surgical techniques or by thoracoscopic surgery techniques. The scaffold structures also lend themselves to delivery to a targeted endocardial or epicardial site by catheter-based techniques. The scaffold structures can serve as templates for the delivery or disbursement of an ablation agent in a prescribed pattern, which forms discrete circumferential lesion patterns in tissue, e.g., tissue surrounding the pulmonary veins. The lesion patterns create conduction blocks for ectopic pacemakers, which can prevent or at least mitigate AF.

Other features and advantages of the invention shall be apparent based upon the accompanying description, drawings, and claims.

### Description of the Drawings

Fig. 1A is a front perspective view of an endocardial heart treatment device that is sized and configured, in use, to rest within a heart chamber and establish a stable platform that makes it possible to accomplish a desired therapeutic and/or diagnostic objective in a targeted fashion.
Fig. 1B is a rear perspective view of the heart treatment device shown in Fig. 1A, showing the array of delivery sites through which an ablation agent can be delivered into or onto tissue to form a desired lesion pattern.
Fig. 2 is a side view of a delivery catheter that carries on its distal end the heart treatment device shown in Figs. 1A and 1B for the purpose of deploying the device in a heart chamber through endovascular access.
Fig. 3 is a perspective anterior view of the inside of a heart in which the heart treatment device shown in Figs. 1A and 1B has been deployed for the purpose of forming a desired pattern of lesions about the pulmonary veins in the left atrium.
Fig. 4 is a perspective anterior view of the inside of a heart after the heart treatment device shown in Fig. 3 has been removed following the formation of the desired pattern of lesions about the pulmonary veins in the left atrium.
Fig. 5 is a side section view of a therapeutic component carried by the heart treatment device shown in Figs. 1A and 1B, showing the micro-ports through which an ablation agent is delivered onto or into tissue.
Fig. 6 is an end section view of the therapeutic component taken generally along line 6-6 in Fig. 5.
Fig. 7 a side section view of the therapeutic component shown in Fig. 5, showing the delivery of ablation agent through the micro-ports onto or into tissue.
Fig. 8 is a side section view of an alternative embodiment of a therapeutic component that can be carried by the heart treatment device shown in Figs. 1A and 1B, showing the presence of a semi-permeable plug in the micro-ports.
Fig. 9 is an end section view of the therapeutic component taken generally along line 9-9 in Fig. 8.
Fig. 10 is a rear perspective view of a heart treatment device of the type shown in Fig. 1B, showing the presence of micro-needles to serve as the delivery sites through which an ablation agent can be delivered into tissue to form a desired lesion pattern.
Fig. 11 is a side section view of a therapeutic component carried by the heart treatment device shown in Fig. 10, showing the micro-needles through which an ablation agent is delivered into tissue.
Fig. 12 is a rear perspective view of a heart treatment device of the type shown in Fig. 1B, showing the presence of needle-less injection sites to serve as the delivery sites through which an ablation agent can be delivered into tissue to form a desired lesion pattern.
Fig. 13 is a side section view of a therapeutic component carried by the heart treatment device shown in Fig. 12, showing the needle-less injection sites through which an ablation agent is delivered into tissue.
Fig. 14A is a rear perspective view of a heart treatment device of the type shown in Fig. 1B, showing the presence of catalytic energy sources to accelerate or enhance the delivery of an ablation agent into tissue to form a desired lesion pattern, the catalytic energy sources being coupled to an external energy source.
Fig. 14B is a perspective anterior view of the inside of a heart in which a heart treatment device of the type shown in Figs. 1A and 1B has been deployed for the purpose of forming a desired pattern of lesions about the pulmonary veins in the left atrium, Fig. 14B also showing the presence of catalytic energy sources to accelerate or enhance the delivery of an ablation agent into tissue, the catalytic energy sources being coupled to an implanted energy generator.
Fig. 15 is a perspective superior view of the inside of a heart in which a heart treatment device of the type shown in Figs. 1A and 1B has been deployed for the purpose of forming a desired pattern of lesions about the pulmonary veins in the left atrium, Fig. 15 also showing the presence of a remote catalytic energy source to accelerate or enhance the delivery of an ablation agent into tissue.
Fig. 16A is a front perspective view of a braced, intra-atrial heart treatment device that is sized and configured, in use, to rest within a heart chamber and establish a stable platform that makes it possible to accomplish a desired therapeutic and/or diagnostic objective in a targeted fashion.
Fig. 16B is a rear perspective view of the heart treatment device shown in Fig. 16A, showing the array of delivery sites through which an ablation agent can be delivered into or onto tissue to form a desired lesion pattern.
Fig. 17 is a perspective anterior view of the inside of a heart in which the heart treatment device shown in Figs. 16A and 16B has been deployed for the purpose of forming a desired pattern of lesions about the pulmonary veins in the left atrium.
Fig. 18 is a perspective anterior view of the inside of a heart after the heart treatment device shown in Fig. 17 has been removed following the formation of the desired pattern of lesions about the pulmonary veins in the left atrium.
Figs. 19A and 19B are front perspective views of a braced, intra-atrial heart treatment device of the type shown in Figs. 16A and 16B, showing the presence of a pull wire that can be operated to alter the shape of the device.
Figs. 20A and 20B are front and rear perspective views of a braced, intra-atrial heart treatment device of the type shown in Figs. 16A and 16B, showing the presence of an auxiliary structure through which an ablation agent can be delivered in a tissue region overlaying the coronary sinus.
Fig. 21 is a perspective anterior view of the inside of a heart in which the heart treatment device shown in Figs. 20A and 20B has been deployed for the purpose of forming a desired pattern of lesions about the pulmonary veins in the left atrium.
Fig. 22 is a perspective anterior view of the inside of a heart after the heart treatment device shown in Fig. 21 has been removed following the formation of the desired pattern of lesions about the pulmonary veins and near the mitral valve annulus in the left atrium.
Figs. 23 to 26 are perspective anterior views of the inside of a heart in which various alternative embodiments of a braced, trans-atrial heart treatment device has been deployed for the purpose of forming a desired pattern of lesions about the pulmonary veins and near the mitral valve annulus in the left atrium.
Figs. 27 to 30 are perspective anterior views of the inside of a heart in which various alternative embodiments of scaffold devices have been deployed, the scaffold devices being sized and configured, in use, to rest within a heart chamber and establish a stable platform that makes it possible to accomplish a desired therapeutic and/or diagnostic objective in a targeted fashion.
Figs. 31 to 33 are perspective anterior views of the inside of a heart in which various alternative embodiments of scaffold devices have been deployed, the scaffold devices being sized and configured, in use, to rest within two heart chambers and establish stable platforms that make it possible to accomplish a desired therapeutic and/or diagnostic objective in a targeted fashion.
Fig. 34 is a side view of an epicardial heart treatment device that is sized and configured, in use, to rest on an epicardial surface of a heart and establish a stable platform that makes it possible to accomplish a desired therapeutic and/or diagnostic objective in a targeted fashion.
Fig. 35 is a perspective anterior views of the outside of a heart on which a device as shown in Fig. 34 has been deployed to encircle the pulmonary veins for the purpose of forming a lesion pattern about the pulmonary veins.

### Detailed Description

Although the disclosure hereof is detailed and exact to enable those skilled in the art to practice the invention, the physical embodiments herein disclosed merely exemplify the invention, which may be embodied in other specific structure. While the preferred embodiment has been described, the details may be changed without departing from the invention, which is defined by the claims.

### I. ENDOCARDIAL HEART TREATMENT DEVICE

### A. General Overview

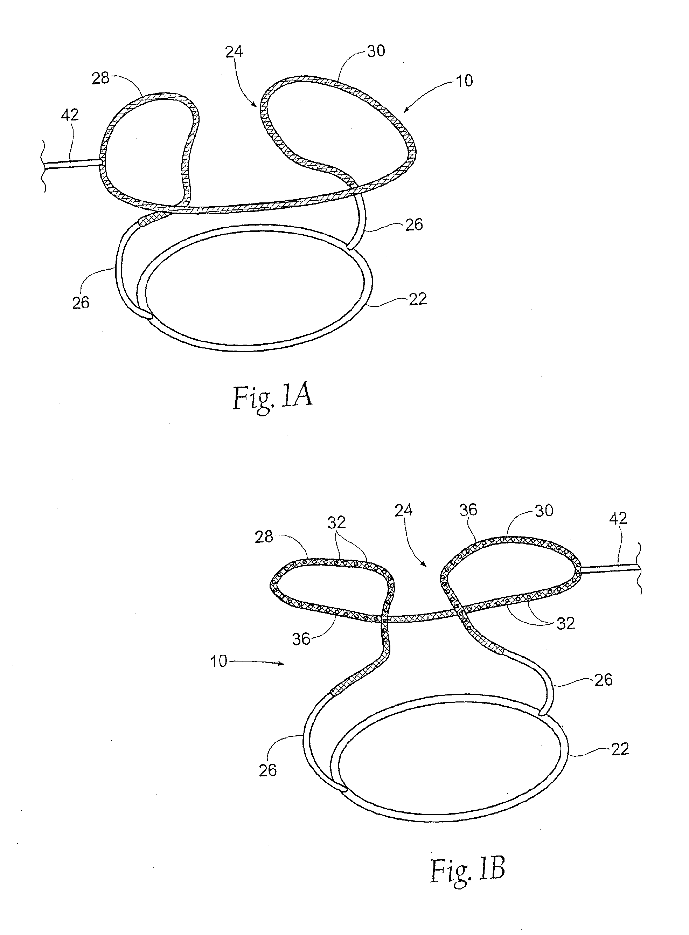

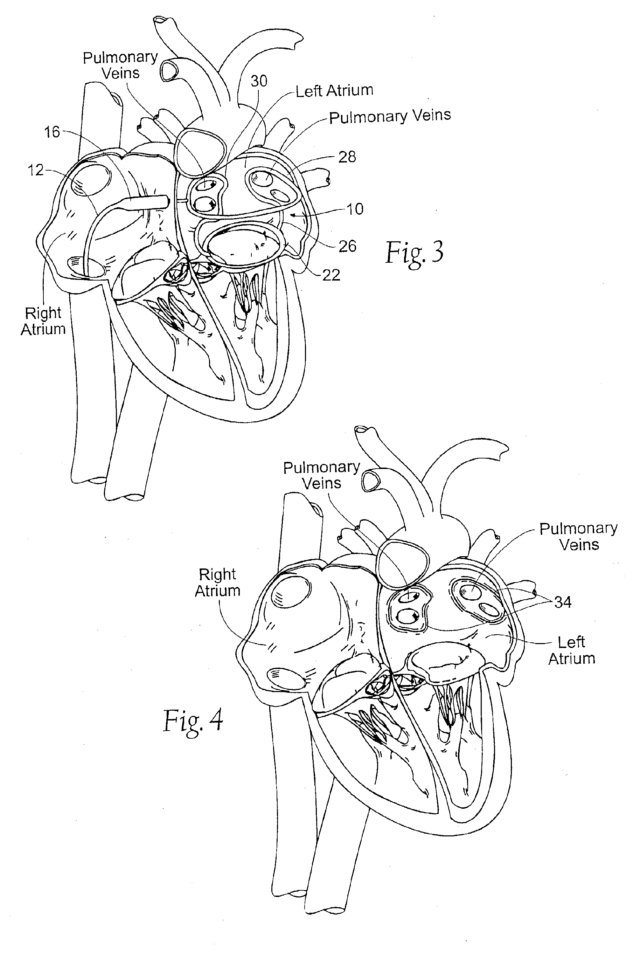

Figs. 1A and 1B show an endocardial heart treatment device 10. The device 10 that is sized and configured, in use, to rest within a heart chamber, as Fig. 3 shows. Fig. 3 shows, as an example, the heart treatment device 10 resting within the left atrium.

The heart treatment device 10 is sized and configured so that, in use, it establishes a stable platform, or scaffold, that makes it possible to accomplish a desired therapeutic and/or diagnostic objective in a targeted fashion in one or more tissue regions. The desired therapeutic and/or diagnostic objectives can include, e.g., (i) the delivery of various chemical, drug, and/or biological agents (in liquid, gel, or wafer form) into contact on or in the tissue regions, to accomplish, e.g., ablation or other therapeutic objectives in a controlled, precise fashion; and/or (ii) the delivery of mechanical, heat, cooling, or electrical energy to the tissue region as a primarily sole therapeutic force or as a catalytic force - e.g., to promote the intake by tissue of a chemical, drug, or biological agent, or to pace heart tissue, or to form temporary or permanent conduction blocks, or to otherwise achieve localized effects in targeted tissue regions such as tissue shrinkage, or combinations thereof; and/or (iii) the positioning of physiologic sensors and/or monitors to analyze heart function for diagnostic purposes, e.g., to ensure wall contact of the device with the atrium by electrically sensing contact.

The endocardial heart treatment device 10 can be made of biocompatible metallic or polymeric materials, or combinations thereof. The device 10 can be deployed within the heart using conventional open heart surgical techniques or by thoracoscopic surgery techniques. As will be described in greater detail later, the heart treatment device 10 can be made from a biocompatible, superelastic metallic material, such as Nitinol™ material, which also lends itself to delivery to a targeted endocardial site by catheter-based, intravascular techniques, guided, e.g., by fluoroscopy, or ultrasound, or combinations thereof. Alternatively, malleable materials such as stainless steel that require re-expansion with a balloon or other expander mechanism after compression for intra-catheter delivery may be used. Fig. 3 shows, by way of illustration, the heart treatment device 10 being introduced by a catheter 12 through the inferior vena cava (from the femoral vein) into the right atrium, and then across the septum between the right and left atriums to a desired position resting in the left atrium

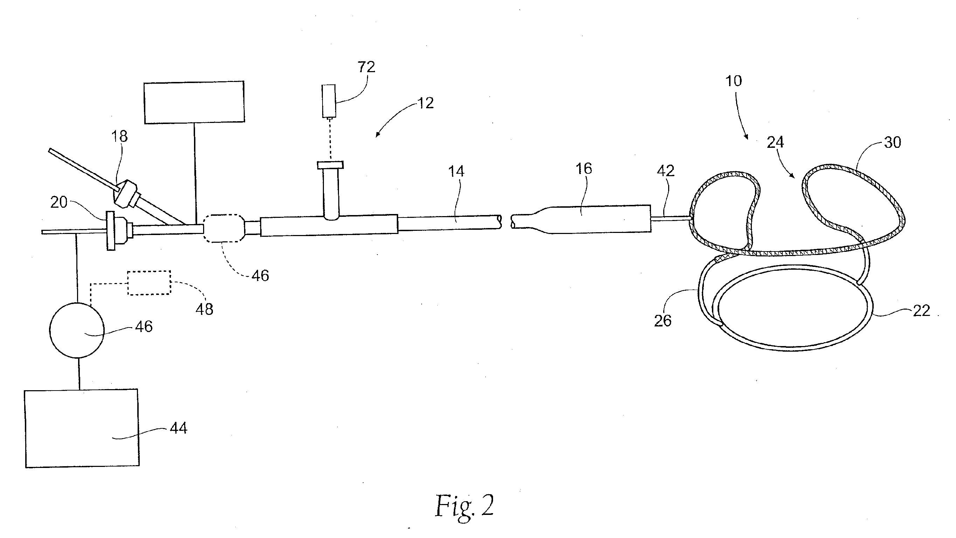

Fig. 2 shows a representative embodiment of a delivery catheter 12, like that generally shown in Fig. 3, for deploying the endocardial heart treatment device 10. The delivery catheter 12 includes a catheter tube 14 that may be, e.g., 12 to 20 French in diameter and up to approximately 150 centimeters long, depending on the patient's anatomy. If the delivery catheter 12 is to be used in a direct surgical approach through the atrial wall to the interior of the left atrium or onto the epicardial surface of the left atrium, lengths close to 50 centimeters would be appropriate.

The heart treatment device 10 is carried on the distal end of the catheter tube 14. In Fig. 2, the device is shown in its fully expanded condition. Typically, the heart treatment device 10 is pre-loaded in a compressed or folded condition within a sleeve 16 on the catheter tube 14 during delivery and expelled from the sleeve 16 to expand *in situ* at the desired destination.

As shown in Fig. 2, the delivery catheter 12 includes a guide wire port 18, as well as a through lumen port 20 for withdrawing or deploying the treatment device into and from the sleeve 16. The catheter 12 is introduced into the patient, typically under fluoroscopic guidance, and advanced through the venous return to the right atrium of the heart. Using standard cardiology procedures, a trans-septal puncture is performed and the catheter 12 is advanced through the trans-septal puncture into the left atrium. Guide wires may be advanced through the guide wire port 18 into the atrial appendage, the mitral valve annulus, and/or one of the pulmonary veins for further guidance. Under continued fluoroscopic guidance, which can also be verified by intravascular ultrasound, the cardiologist will deploy the device 10 from the sleeve 16. The cardiologist will ensure that the device 10 has expanded completely and is appropriately seated in contact in a correct position with the surrounding heart wall, e.g., through the use of a pull wire attached to the device 10 that is manipulated outside the patient by the cardiologist. As will be described in greater detail later, additional seating mechanisms can be used.

The heart treatment device 10 (or any one of the alternative embodiments to be disclosed later) may be used as a temporary platform, or scaffold, for the delivery of a desired therapeutic and/or diagnostic result, after which the heart treatment device 10 is withdrawn. Alternatively, the heart treatment device 10 (or any one of the alternative embodiments disclosed later) may be left in place as a more permanent implant to achieve chronic treatment objectives. For example, a more permanent implant could perform drug elution over time.

The heart treatment device 10 may be variously constructed. In the embodiment shown in Figs. 1A,1B, and 2, deployment within an atrial heart chamber is contemplated. In this embodiment, the heart treatment device 10 includes an annular base 22. The base 22 is sized and configured to rest on or near the inferior valve annulus of the atrial chamber, which in Fig. 3 is the mitral valve annulus of the left atrium. The base 22 provides a foundation to support and stabilize the entire device 10.

As shown in Figs. 1A, 1B, and 2, the base 22 supports and stabilizes at least one component 24 of the device which performs a therapeutic and/or diagnostic function. In the embodiment shown in Figs. 1A, 1B, and 2, the component 24 takes the form of a loop section, which is coupled by upright members 26 to the base 22. In this embodiment, the loop section 24 is intended, in use, to perform the therapeutic function of tissue ablation, and, in particular, the ablation of tissue regions within the left atrium surrounding the pulmonary veins. The pulmonary veins are common sources of ectopic pacemakers. The ablation function of the loop section 24 creates conduction blocks around all four sets of normally occurring pulmonary veins.

To perform this desired function, the loop section 24 is preformed with a curvilinear, figure-8 configuration, which forms two adjoining loops, respectively left loop 28 and right loop 30. In Fig. 3, the loops 28 and 30 are shown to rise from the base 22 into the left atrium and, in use, come into close proximity with tissue that surrounds the left and right pulmonary veins.

The use of a superelastic metal, such as Nitinol™ material, for the base 22 and loop section 24 makes possible the formation of specific shapes when the device 10 is deployed. The shapes possess specific outward-going or hoop strengths, which exert force against a tissue wall when the heart treatment device 10 is deployed and reaches a given size. The characteristics of a superelastic metal like Nitinol™ material are also useful, as the heart treatment device 10 will reliably assume a pre-shaped configuration when released from a delivery catheter. In this arrangement, guide wires may be introduced into each set of left and right pulmonary veins to facilitate placement of the loops 28 and 30.

### B. Delivery of Ablation Agent

As Fig. 1B shows, the loops 28 and 30 each carries an array of spaced-apart sites 32 for delivering or dispersing an ablation agent in liquid or gel form into or onto tissue. The delivery sites 32 are located along the mural facing surface of the loops 28 and 30, so that the ablation agent is preferentially released onto or into adjacent tissues, and not into the blood volume of the heart chamber. In additional, a remotely actuated cover mechanism carried on the loops 28 and 30 can be used to direct the release of ablation energy away from the blood stream.

As Fig. 4 shows, the loops 28 and 30 serve as templates for the delivery or disbursement of the ablation agent in a prescribed pattern, which forms discrete circumferential lesion patterns 34 in tissue surrounding the pulmonary veins. The lesion patterns 34 create conduction blocks for ectopic pacemakers, which can prevent or at least mitigate AF. The conduction blocks are preferably fully transmural through the myocardium of the atrium (about 3 to 5 millimeters in thickness). Once the conduction block has been completed, the device 10 may be removed from the patient, as Fig. 4 shows.

The structure and mode of mode of operation of the ablation agent delivery sites 32 can vary.

### 1. -Ports

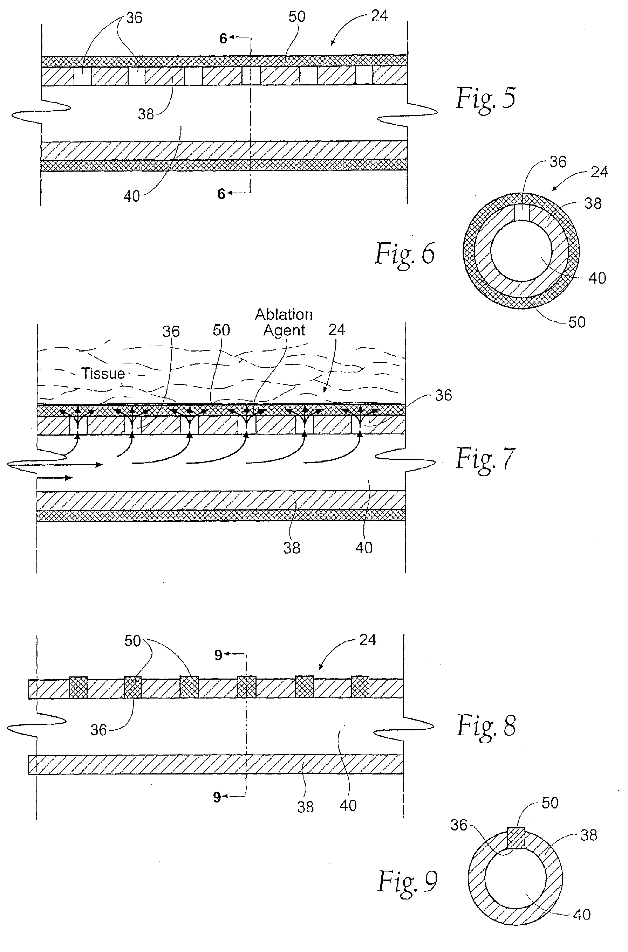

In the embodiment illustrated in Fig. 2, the sites comprise an array of micro-ports 36, through which the ablation agent is conveyed. In this arrangement (see Figs. 5 and 6) the loop section 24 defines a tubule 38 (desirably made of Nitinol™ material) that peripherally defines an interior passage 40. The interior passage 40 is continuous through the two loops 28 and 30. The micro-ports 36 are cut, e.g., by laser, into the tubule 38, forming the array of delivery sites 32 that communicate with the interior passage.

The interior passage 40 communicates with an agent delivery tube 42 (see Figs. 1A, 1B, and 2). The tube 42 passes through the deployment catheter 12 (see Fig. 2) to an external source 44 of the ablation agent. The tube 42 conveys the ablation agent into the interior passage 44 under pressure by use of a suitable pumping mechanism 46, e.g., a syringe pump, or a peristaltic pump, or a diaphragm pump, or a suitable piezoelectric pump. The pump mechanism 46 may be external (as Fig. 2 shows in solid lines), or it may comprise a pumping mechanism carried in-line in the catheter tube (as Fig. 2 shows in phantom lines).

Alternatively, the pumping mechanism 46 can comprise a mechanical displacement mechanism, e.g., a piston chamber, may be used to pump the ablation agent under pressure for disbursement through the micro-ports 36. In this embodiment, the displacement mechanism of the pumping mechanism 46 can accept a compressed gas canister or vacutainer 48 (shown in phantom lines in Fig. 2) to drive the piston mechanism. A release button controls the insufflation of the compressed gas into the piston chamber, which expels the ablation agent through the tube 42 into the interior passage 40. The mechanically pressurized agent is forcibly delivered into or onto the tissue. Other propulsion gases or mechanical piston activators or non-piston fluidic jet propulsion mechanisms may also be applied.

Regardless of the particular form and function of the pumping mechanism 46, the ablation agent is dispensed (when in liquid form) or extruded (when in solid or semi-solid gel form) through the micro-ports 36 into or onto tissue.

It should be appreciated that, when tubule 38 is formed from a Nitinol™ material, the hoop strength of the tubule 38 can be adjusted by controlling the temperature of the ablation agent - or whatever fluid that is conveyed into the interior passage. Thus, the magnitude of the force exerted by a given tubule 38 against a tissue wall can be governed by heating or cooling.

Optionally (see Fig. 7), a semi-permeable material 50 may be used to gate disbursement of the ablation agent through each micro-port 36. The semi-permeable material 50 can comprise a polymeric material made, e.g., from sintered gel or hydrogel formulation. The porosity of the semi-permeable material 50 is sized and configured to meter the release the ablation agent. The semi-permeable material 50 desirably functions as a diffusion barrier. As Fig. 7 shows, when the ablation agent is released from the micro-ports 36, the semi-permeable material 50 slows the rate of flow and promote an equal distribution of fluid from the loop section 24. The ablation agent diffuses from the semi-permeable material 50 into or on adjacent heart tissue, rather than being released like a jet into the surrounding heart tissue.

In the embodiment shown in Figs. 5 and 6, the semi-permeable material 50 takes the form of a sleeve. The sleeve surrounds the tubule 38 and overlays the micro-ports 36.

In the embodiment shown in Figs. 8 and 9, the semi-permeable material 50 takes the form of a plug that occupies each micro-port. The plug can be made, e.g., from sintered gel beads formed into a porous structure.

### 2. Micro-Needles

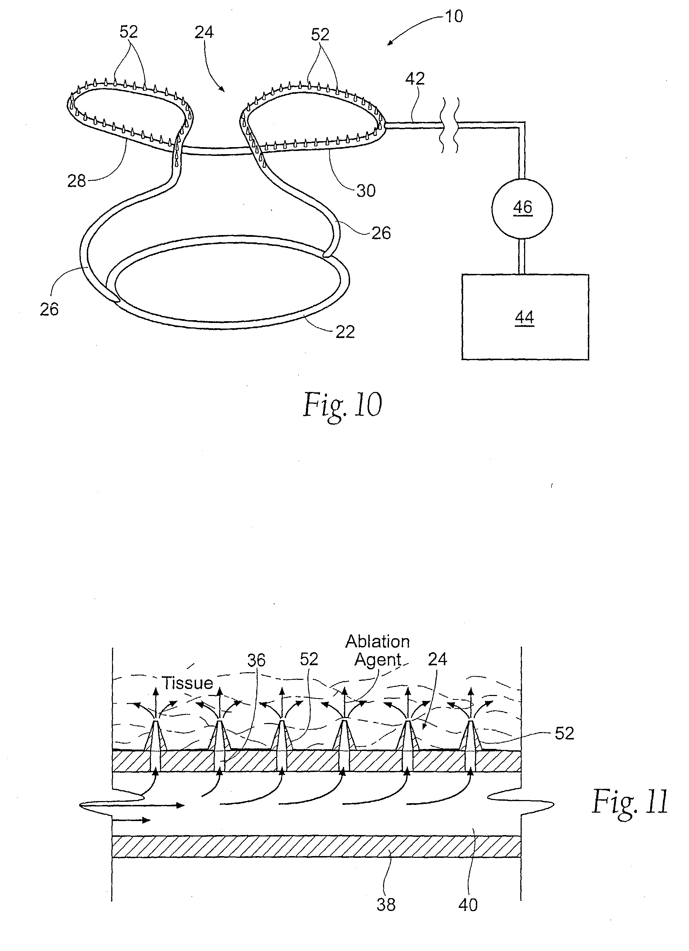

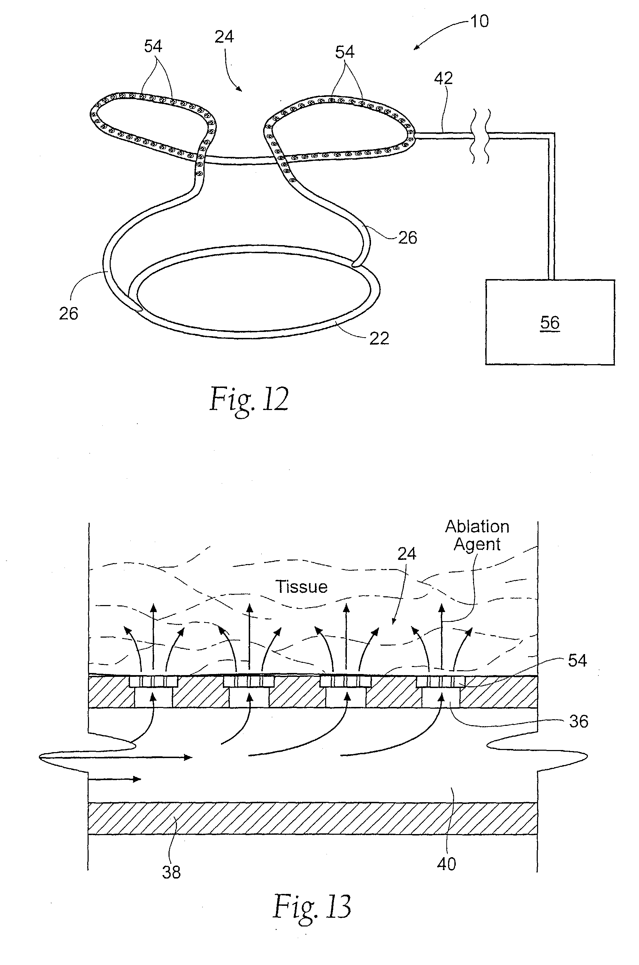

In the alternative embodiment illustrated in Figs. 10 and 11, the sites 32 each comprise a micro-needle 52 carried on the tubule 38 in communication with the interior passage 40. As Fig. 11 shows, upon deployment of the device 10, the micro-needles 52 penetrate tissue in the pattern that the loop section 24, serving as a template, creates. The ablation agent pumped (via the pumping mechanism 46) into the interior passage 40 is preferentially injected through the micro-needles 52 into tissue, to form the lesion patterns 34 shown in Fig. 3. It should be appreciated that a device 10 can incorporate an array of micro-needles 52 in combination with an array of micro-ports 36 as already described.

### 3. Needle-Less Injection

In the alternative embodiment illustrated in Figs. 12 and 13, the sites each comprises an array of small diameter (e.g., 0.00025" to 0.020"), needle-less injection ports 54. In this arrangement, the ablation agent is conveyed into the interior passage 40, while a relatively high pressure-control source 56 (e.g., upward to about 300 psi) conveys periodic bursts of elevated pressure into the interior passage 40. As Fig. 13 shows, the pressure bursts propel the ablation agent from the ports 54 as fluid jets or streams capable of penetrating tissue disposed in contact with or adjacent to the loop section 24. The ablation agent is injected in the pattern that the loop section 24, serving as a template, creates. As a result, the lesion patterns 34 shown in Fig. 3 are formed. It should be appreciated that a device 10 can incorporate an array of micro-needles 52 and/or an array of needle-less injection ports 54 in combination with an array of micro-ports 36 as already described.

### C. The Ablation Agent

The ablation agent selected for use can vary.

The ablation agent may include tissue fixatives like alcohol, ethanol, DMSO, or acetone in liquid form. Alternatively, the ablation agent may comprise hydrogel formulations of tissue fixative agents impregnated within solid and semi-solid gels. In this arrangement, the treatment device may serve to passively maintain the hydrogel in contact with the tissue long enough to achieve the desired effect, or may detach from the hydrogel and leave it in tissue contact sufficiently long to allow the ablation agent to permeate the tissue. The hydrogel carrier may completely dissolve or be retrieved from the tissue site with withdrawal of the device.

The benefits of using alcohol, or another tissue fixative agent, include the significant reduction or elimination of the amount of energy required to create conduction block. This results in a safer and more effective ablation, because the tissue is in fact toughened by the fixative properties of alcohol-like agents that cause a coagulation cellular necrosis. This is in contrast to an initially weakened tissue wall liquefaction necrosis, which is often associated with perforation through the wall or into adjacent contiguous structures caused by using heat-creating energy forms to create the conduction block. Use of a tissue fixative creates an ablation without risk of perforation associated with the ablation agent, in comparison to the liquefaction necrosis associated with heat energy therapies, or risk of inadequate non-transmural effects, or risk of stenosis of a pulmonary vein caused by heat.

Alternatively, the ablation agent can include a tissue stain like potassium iodide solutions; or therapeutic drugs, etc. in liquid form. Therapeutic drug formulations can include anti-arrhythmia drugs, antimetabolites, and/or genetic materials to cause ablation. Alternatively, the ablation agent may comprise hydrogel formulations of therapeutic drug or genetic material impregnated within solid and semi-solid gels. The ability to locate and stabilize the preformed loop section makes possible the delivery of anti-arrhythmia drugs in a targeted fashion to temporarily "reprogram" the electrical characteristics of a localized tissue region or regions. The ability to locate and stabilize the preformed loop section also makes possible the delivery of genetic materials in a targeted fashion to affect permanent change in the electrical characteristics of a localized tissue region or regions.

### D. Catalytic Energy Sources

By use of pressure-driven application of the ablation agent and/or the use of micro-needles 52, the establishment of intimate contact between the delivery sites 32 and tissue is not absolutely essential. Gap or gaps of incomplete contact between the device 10 and the targeted tissue regions may exist, without significantly degrading the therapeutic objective of ablation.

As an adjunct to delivery of the ablation agent in liquid or gel form, the endocardial heart treatment device 10 may rely upon the localized application of mechanical energy, heat energy, and/or electrical energy, and/or the dispensement an auxiliary agent in liquid or gel form, to condition the tissue to increase or accelerate the intake of the ablation agent. Many types of catalytic energies or agents can be delivered to the targeted tissue region concurrent with the delivery of the ablation agent, e.g., ultrasound, radiofrequency microwave, electrophoresis, or resistor-based electric heat. These catalytic energies encourage the flow of ions in a preferred direction, and/or encourage fluid absorption, and/or cause ablation to occur. The ancillary application of such energy causing, e.g., electroporation or sonoporation or heat, disrupt native tissue physiology sufficient to enhance the baseline effectiveness of primary treatment mechanism, generating catalytic mechanisms such as electrophoresis or sonophoresis or cell death that make the tissue more receptive to the ablation agent.

As Fig. 14A shows, one or more catalytic energy sources 58 may be carried directly on the device 10 itself. The base 22 or the loop section 24 of the device 10 may include at least one catalytic energy source 58, or provide for the delivery of at least one catalytic agent, to accelerate delivery of the ablation agent into the tissue. For example, the base 22 and/or loop section 24 can carry as a catalytic energy source 58, e.g., at least one radiofrequency-emitting electrode, and/or ultrasonic micro-crystal, and/or ultrasonic micro-transducer, and/or a wave propagation device including wires and ultrasonic horns, and/or another device that propagates mechanical, heat, or ultrasonic energy, which can employ, e.g., micro electromechanical system (MEMS) technologies. The spacing between these energy sources 58 on the device 10 takes into account the propagation patterns of the energy itself. For example, taking into account that ultrasound travels in straight lines as rapidly decreasing energy, the distances between ultrasound crystals need to be on the order of every 1 mm to 10 mm in target range along the course of the base or loop section.

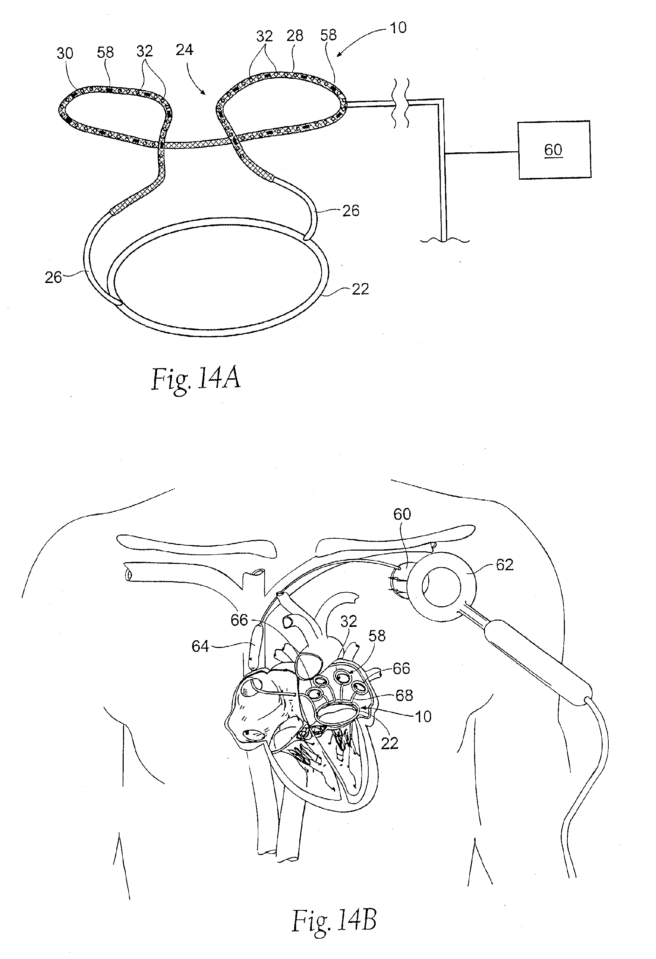

The catalytic energy sources 58 are coupled to energy generation devices 60 (see Fig. 14A). Energy generation devices 60 can, as Fig. 14A shows) be positioned external to the body and be coupled by wires to the energy sources 58 carried on the device 10. The energy generation devices 60 can also implanted within the body. For example, as Fig. 14B shows, an energy generation device 60, such as an ultrasound or radio frequency generator, can be implanted underneath the skin (subclavian pocket), and communicate with a device 10 located in the left atrium by way of the superior vena cava via the subclavian vein. The energy generation device 60 can receive power transcutaneously from a remote activator 62. The energy generation device 58 may also include an implanted, in-line control component 64, e.g., implanted within the superior vena cava as Fig. 14B shows, which may function as a transformer, capacitor, or electrode.

It should also be noted that the size and configuration of the endocardial treatment device shown in Fig. 14B differs from that shown in Figs. 1A and 1B. This is done to illustrate that the device can be variously constructed and configured. In Fig. 14B, the annular base 22 supports four individual therapeutic tubules, each formed as a loop 66. The loops 66 are separately coupled by upright members 68 to the base 22. Each loop 66 includes an array of agent delivery sites 32, as well as catalytic energy sources 58, which are coupled to the energy generation device 60.

Alternatively, the catalytic energy sources 58 may be located remote of the device 10, and without direct contact with the targeted tissue region, e.g., they can be located trans-esophageally, trans-bronchially, transtracheally, trans-thoracically, across the sternum, etc.

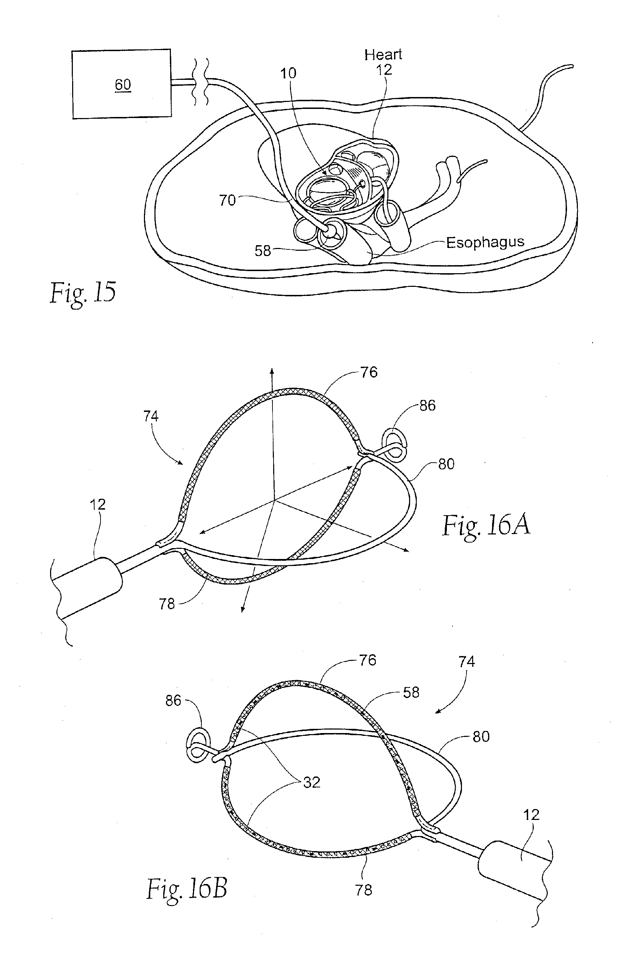

Fig. 15 shows, by way of illustration in a schematic view, a patient with a catheter 12 advanced from the inferior vena cava, into the right atrium, and across the septum into the left atrium. The catheter 12 deploys the heart treatment device 10. A second catheter 70 is advanced through the esophagus. The second catheter 70 carries a catalytic energy source 58, such as ultrasound (preferably low frequency ultrasound, below 1 MHz), and/or radio frequency, and/or an inductive coupling mechanism, and/or microwave. The energy source applies 58 the catalytic energy to the left atrium to enhance the delivery of ablation agent by the first catheter 12. Alternatively, ultrasound and other catalytic energy sources 58 may be delivered to the left atrium transcutaneously across the skin.

The delivery of catalytic energy as an adjunct to the delivery of an ablation agent, requires a significantly lesser amount of energy than is typically required when thermal tissue ablation alone is desired. This improved energy utilization efficiency is achieved by using the energy source, not as the primary ablation agent, but as a catalyst for a drug, chemical or biological agent. This aspect improves both the efficacy and safety of ablation in terms of unwanted tissue destruction and collateral damage. Furthermore, the catalytic energy sources 58 as described can be embodied in low power devices, and can be coupled for power to, e.g., a battery 72 that can be inserted into the delivery catheter 12 (see Fig. 2), to render the overall system totally contained and disposable.

In an alternative arrangement, the catalytic energy sources 58 could themselves be used to perform ablation without the use of an ablation agent, independent of or in combination with their catalytic function.

### E. Other Representative Embodiments

The endocardial heart treatment device 10 can sized and configured in different ways to possess different mechanical properties.

### 1. Braced, Intra-Annular Devices

For example, in Figs. 16A and 16B, a heart treatment device 74 includes a superior curvilinear tubule 76 (superior relative to the pulmonary veins), an inferior curvilinear tubule 78 (inferior relative to the pulmonary veins), and a hemispherical brace frame 80. The brace frame 80 extends in a geometrically opposing relationship relative to the superior and inferior tubules 76 and 78.

As Fig. 16B shows, the superior and inferior tubules 76 and 78 each includes an array of delivery sites 32 for delivering or dispersing an ablation agent in liquid or gel form into or onto tissue. The delivery sites 32 are located along the mural facing surface of the tubules 76 and 78, so that the ablation agent is released onto or into adjacent tissues and not into the blood volume of the heart chamber. In additional, a remotely actuated cover mechanism carried on the tubules 76 and 78 can be used to direct the release of ablation energy away from the blood stream.

The tubules 76 and 78 form templates for the delivery or disbursement of the ablation agent, which forms discrete circumferential lesion patterns in tissue surrounding the pulmonary veins. The delivery sites 32 can comprise micro-ports, micro-needles, needle-less injection sites, or combinations thereof, as already described.

As Fig. 16B also shows, the superior and inferior tubules 76 and 78 can also each carry one or more catalytic energy sources 58, as already described, to accelerate delivery of the ablation agent into the tissue.

The superior and inferior tubules 76 and 78 and the brace frame 80 shown in Figs. 16A and 16B desirably incorporate superelastic metal like Nitinol™ material, as previously described. This makes possible the formation of specific shapes when the device 74 is deployed. The shapes possess specific outward-going or hoop strengths, which exert force against a tissue wall when the heart treatment device 74 is deployed and reaches a given size. The characteristics of a superelastic metal like Nitinol™ material are also useful, as the heart treatment device 74 will assume a pre-shaped configuration when released from a delivery catheter. The use of superelastic metallic material, such as Nitinol™ material, which also lends itself to delivery to a targeted endocardial site by catheter-based, intravascular techniques previously described.

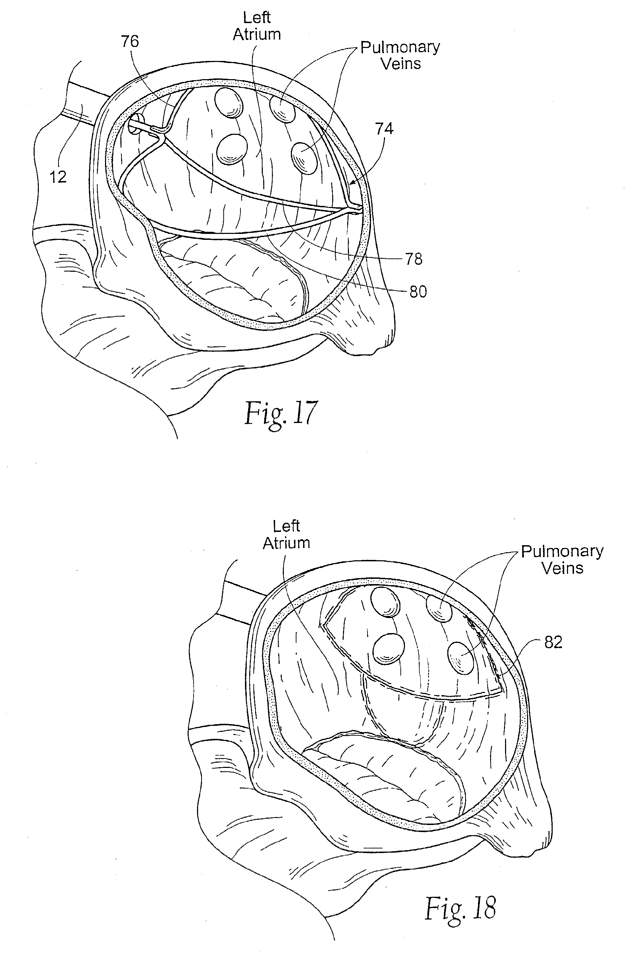

Fig. 17 shows the heart treatment device 74 being introduced by a catheter 12 through the inferior vena cava (from the femoral vein) into the right atrium, and then across the septum between the right and left atriums to a desired position resting in the left atrium.

When deployed in the left atrium for use (assuming the trans-septal introduction just described, as Fig. 17 shows), the proximal end of the device 74 is positioned adjacent the trans-septal entry point. The brace frame 80 is positioned along the anterior wall, opposite the pulmonary veins. The brace frame 80 functions to transmit mural pressure from the atrium through the device to the superior and inferior tubules 76 and 78. The superior tubule 76 is braced by the brace frame 80 into position adjacent the apex of the left atrium. The inferior tubule 78 is braced by the brace frame 80 into position adjacent the base of the posterior wall. The delivery sites 32 are placed into contact with tissue along these positions. The brace frame 80 drives the delivery sites 32 firmly against the tissue wall to ensure integrity of contact along the course of the tubules 76 and 78.

The delivery sites 32 release an ablation agent from inside the tubules 78 and 80 and onto or into the atrial walls, to create an electrical conduction block 82. As Fig. 18 shows, the shape of the conduction block 82 created by the device 74 is that of an oval, or a football, or a stellate configuration which bow above and below the entire set of four pulmonary veins as a unit. The lines follow a path similar to two adjacent longitudinal lines on a world globe (turned sideways) beginning at the North Pole, and ending at the South Pole. In this arrangement, ablation occurs far enough away from the pulmonary veins so as not to create stenosis. No pulmonary vein itself is ablated. Desirably, the tubules 76 and 78 are no less than 5 mm away from any pulmonary vein, but ideally within 25 mm of any pulmonary vein.

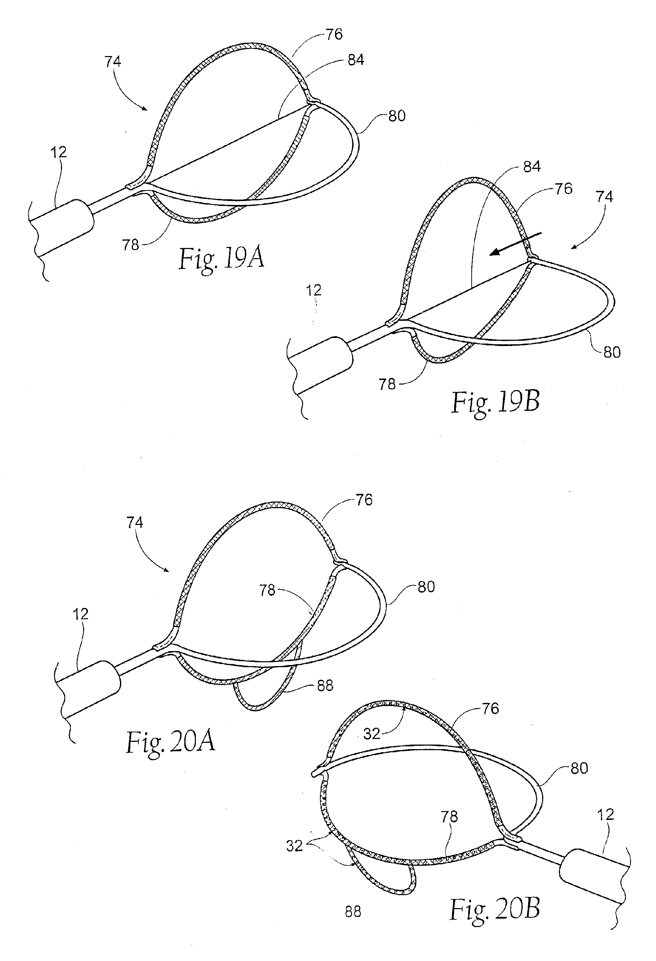

The hoop strength of the device 74 may be adjusted by partially withdrawing or extending the device from the catheter 12. The device 74 may also include a pull wire 84 coupled to the distal end (see Figs. 19A and 19B). In this arrangement, by pulling the pull wire 84 aft (keeping the catheter 12 stationary) (see Fig. 19B), the brace frame 80 can be forced toward a more pronounced outwardly bowed condition. This establishes firmer contact with the endocardium, while increasing the general diameter of the tubules 76 and 78, to better control the contact forces between the device 74 and the atrial wall.

The device 74 may also include an orienting structure 86 on its distal end (see Figs. 16A and 16B). The orienting structure 86 is sized and configured to fit into an anatomic location within the heart chamber, to achieve proper orientation of the device 74 within a heart chamber. As shown in Figs. 16A and 16B, the orienting structure 86 takes the form of a circular frame projecting from the distal end of the device 74. In use, the circular projection can be positioned, e.g., within an atrial appendage, to aid with orientation of the device 74. The orienting structure 86 may also take the shape of a pigtail, or corkscrew projecting from the distal end of the device 74.

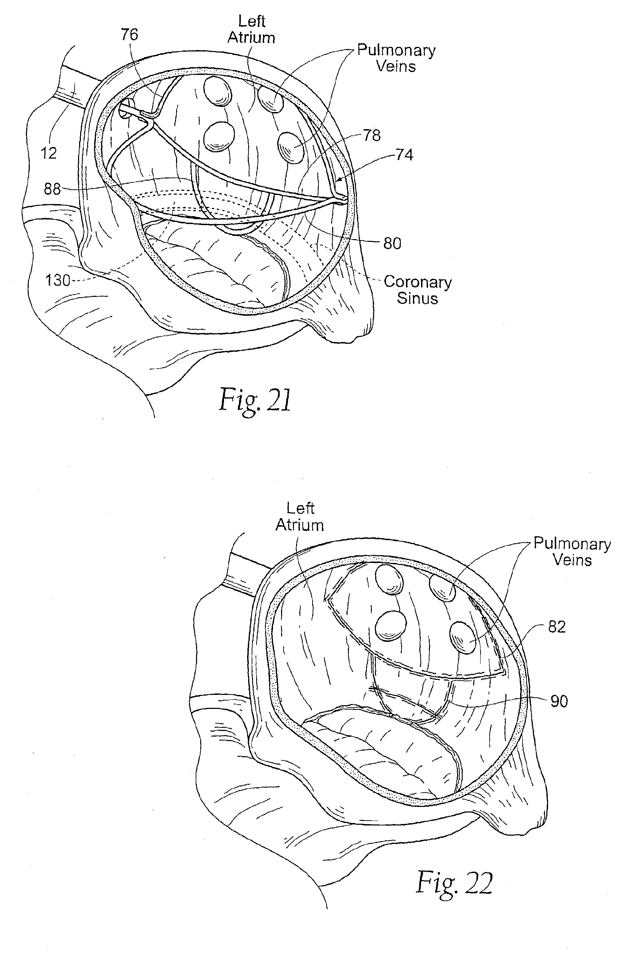

In addition (as Figs. 20A and 20B show), the device 74 can be designed with one or more additional therapeutic tubules 88 to create additional lines of ablation. For example, as shown in Figs. 20A and 20B, the device 74 can include an auxiliary therapeutic tubule 88 appended to the inferior therapeutic tubule 78, which is sized and configured to cross the coronary sinus and loop toward and across the posterior mitral valve annulus (see Fig. 21). The auxiliary tubule 88 includes delivery sites 32 to deliver an ablation agent along this path, which overlays a portion of a coronary sinus and a portion of a posterior mitral valve annulus. As Fig. 22 shows, this makes possible the formation of a lesion pattern 90 in the left atrium where the coronary sinus lies outside the left atrium. Thus (as Fig. 21 shows), a operator such as a cardiologist or cardiac surgeon can deploy an ablation catheter 130 into the coronary sinus to ablate tissue within the coronary sinus, as the device 74 is being used to perform ablation about the pulmonary veins and across at least one region of the posterior annulus. The coronary sinus is desirably ablated circumferentially across the auxiliary tubule 38 (as Fig. 22 shows), to complete the electrophysiological isolation of the pulmonary veins. Additional non-therapeutic base frames may also be provided. Positioning can be facilitated by imaging technologies such as transesophageal echo, intracardiac echo, fluoroscopy, MRI imaging.

The heart treatment device 74 may be used as a temporary platform, or scaffold, for the delivery of a desired therapeutic and/or diagnostic result, after which the heart treatment device 74 is withdrawn. Alternatively, the heart treatment device 74 may be left in place as a more permanent implant to achieve chronic treatment objectives. For example, a more permanent implant could perform drug elution over time.

### 2. Septum Anchored and Annulus Braced, Trans-Atrial Devices

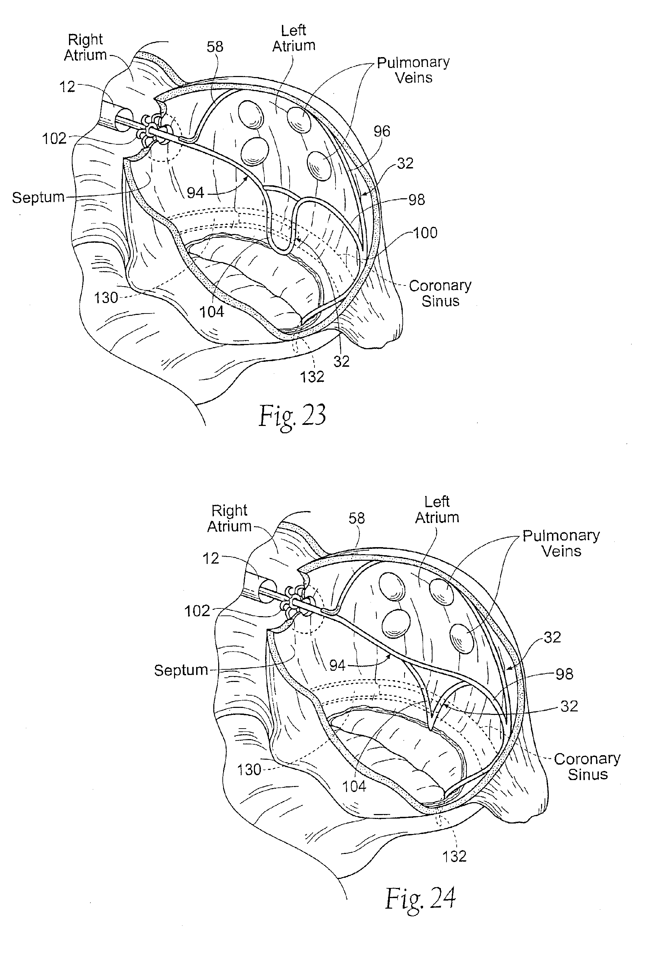

Fig. 23 shows another embodiment of an endocardial heart treatment device 94. The device 94 includes a superior curvilinear tubule 96 (superior relative to the pulmonary veins) and an inferior curvilinear tubule 98 (inferior relative to the pulmonary veins). The device 94 also includes a distal frame 100 that extends laterally beyond the superior and inferior tubules 96 and 98. The frame 100 is sized and configured to contact the lateral wall of the left atrium. As Fig. 23 shows, the frame 100 can include a strut 132 that extends through the lateral annulus into the ventricle for additional support. Braced on the distal end by the frame 100 against the lateral wall, and braced on the proximal end by an anchor 102 in the septum in the right atrium, the superior and inferior tubules 96 and 98 are held in compression against or near the posterior wall of the left atrium above and below the pulmonary veins. A pair of oppositely facing anchors 102 can be placed in the septum in both the left and right atriums to provide further leverage and bracing to push the frame 100 against atrial tissue, to ensure contact of the superior and inferior tubules 96 and 98 in compression against or near the posterior wall of the left atrium above and below the pulmonary veins.

The device 94 also includes an auxiliary therapeutic tubule 104 appended to the inferior therapeutic tubule 98, which is sized and extended to extend down across the coronary sinus and toward and across the posterior mitral valve annulus (as Fig. 23 shows). The therapeutic tubule 104 is thereby sized and configured to rest adjacent to tissue on the posterior wall and overlay a portion of a coronary sinus and a portion of a posterior mitral valve annulus.

The superior and inferior tubules 96 and 98 each includes an array of delivery sites 32 positioned against or near the posterior wall of the left atrium for delivering or dispersing an ablation agent in liquid or gel form into or onto tissue. The auxiliary tubule 104 also includes delivery sites 32 to deliver an ablation agent along this path.

The delivery sites 32 are located along the mural facing surface of the tubules 96, 98, and 104 so that the ablation agent is released onto or into adjacent tissues and not into the blood volume of the heart chamber. In additional, a remotely actuated cover mechanism carried on the tubules 96, 98, and 104 can be used to direct the release of ablation energy away from the blood stream.

The superior and inferior tubules 96 and 98 and the distal frame 100 shown in Fig. 23 desirably incorporate superelastic metal like Nitinol™ material, as previously described. This makes possible the formation of specific shapes when the device 94 is deployed. The shapes possess specific outward-going or hoop strengths when placed into compression, which exert force against a tissue wall when the heart treatment device 94 is deployed and reaches a given size. The use of superelastic metallic material, such as Nitinol™ material, which also lends itself to delivery to a targeted endocardial site by catheter-based, intravascular techniques previously described.

As before explained, the tubules 96 and 98 form templates for the delivery or disbursement of the ablation agent, which forms discrete circumferential lesion patterns in tissue surrounding the pulmonary veins and across the posterior annulus. The coronary sinus is desirably ablated circumferentially across the auxiliary tubule 104 (in the manner shown in Fig. 22) using an ablation element 130 disposed in the coronary sinus, to complete the electrophysiological isolation of the pulmonary veins. The lesion patterns formed by the device 94 shown in Fig. 23 follow generally the same oval shape as shown in Fig. 22, with an auxiliary lesion pattern across the posterior annulus and in the left atrium where the coronary sinus lies outside the left atrium.

The delivery sites 32 can comprise micro-ports, micro-needles, needle-less injection sites, or combinations thereof, as already described. The superior and inferior tubules 96 and 98 and/or auxiliary tubule 104 can also each carry one or more catalytic energy sources 58, as already described, to accelerate delivery of the ablation agent into the tissue.

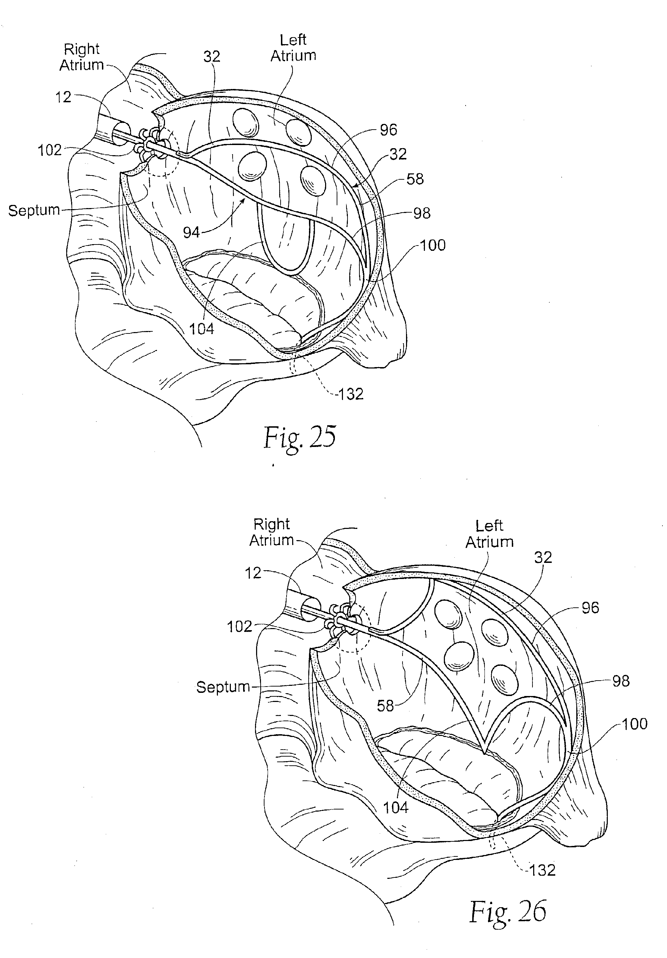

Figs. 24, 25, and 26 show other illustrative embodiments of trans-atrial devices 94 that, like the device shown in Fig. 23, are braced on the distal end by a frame 100 against the lateral wall (with a strut 132 that crosses the annulus into the ventricle), and braced on the proximal end by an anchor 102 in the septum in the right atrium, to hold the superior and inferior tubules 96 and 98 and auxiliary tubule 104 in compression against or near the posterior wall of the left atrium above and below the pulmonary veins.

Any one of the heart treatment devices 94 may be used as a temporary platform, or scaffold, for the delivery of a desired therapeutic and/or diagnostic result, after which the heart treatment device 94 is withdrawn. Alternatively, any one of the heart treatment devices 94 may be left in place as a more permanent implant to achieve chronic treatment objectives. For example, a more permanent implant could perform drug elution over time.

### 3. Other Scaffold Devices

The fundamental concept of using a superelastic scaffold structure within the heart or a body cavity, to establish a desired pattern of tissue contact to serve as a template for the delivery a liquid or gel or electromagnetic ablation agent, can be exemplified in other forms and structures. The concept is not confined to devices that necessarily create lesions about the pulmonary veins, but the scaffold structure can be deployed to support monitoring devices and/or sensing devices and/or medical delivery devices and/or other types of therapeutic/diagnostic apparatus. Representative embodiments of such scaffold devices will be described for purposes of illustrating this point.

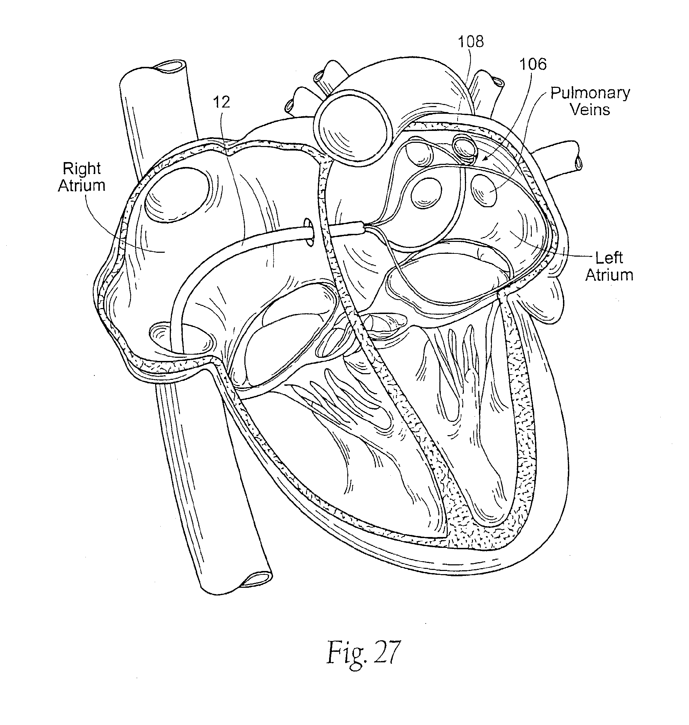

Fig. 27 shows a representative scaffold device 106 that, when deployed, forms a cage. The scaffold device 106 includes a positioning structure 108 to standardize device orientation within a treated heart chamber. In this embodiment, the positioning structure 108 is shown resting in a pulmonary vein to orientate the remainder of the device 106.

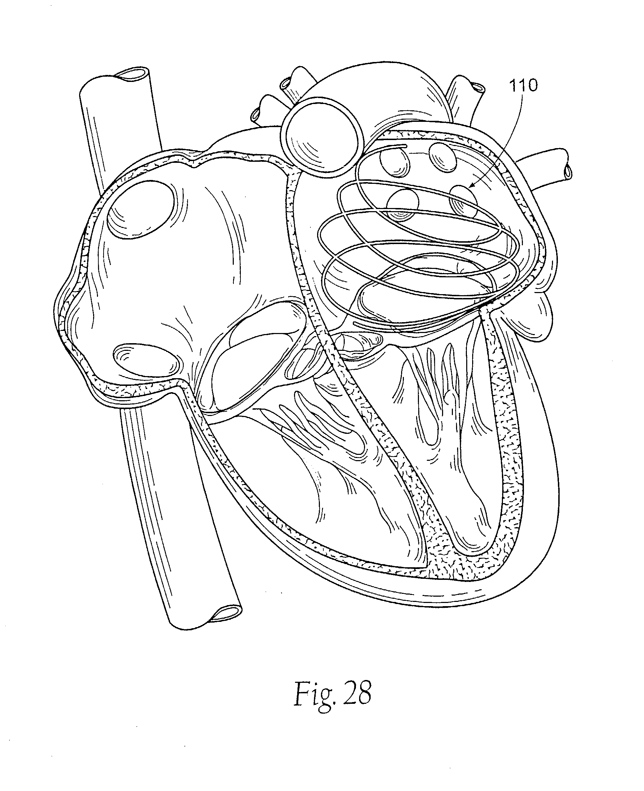

Fig. 28 shows another representative scaffold device 110 in the form of a wire coil that, when deployed, closely conforms to the interior of a patient's heart chamber, which is, in Fig. 28, the left atrium. The deployed scaffold device 110 has an approximately cylindrical configuration. The wire coil of the scaffold device 110 may be constructed of a malleable or elastic biocompatible metal, such as stainless steel or a superelastic or shape memory nickel/titanium alloy, for example. Preferably, the scaffold structure 110 is sufficiently flexible such that it does not interfere excessively with the normal contraction of the heart. In addition, the wire coil may have a coating for improved biocompatibility, thermal and/or electrical insulation, etc.

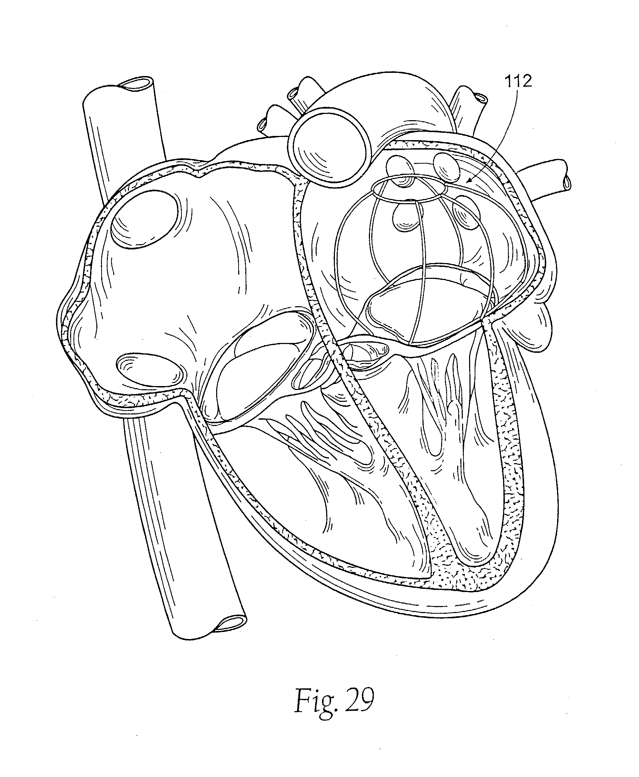

Fig. 29 shows another representative embodiment for a scaffold device 112. The scaffold device 112 is in the form of a wire cage that, when deployed, closely conforms to the interior of a patient's heart chamber, such as the patient's left atrium. The deployed scaffold structure 112 may have a dome-shaped or tapered cylindrical configuration, with an upper loop and a lower loop joined by longitudinal struts.

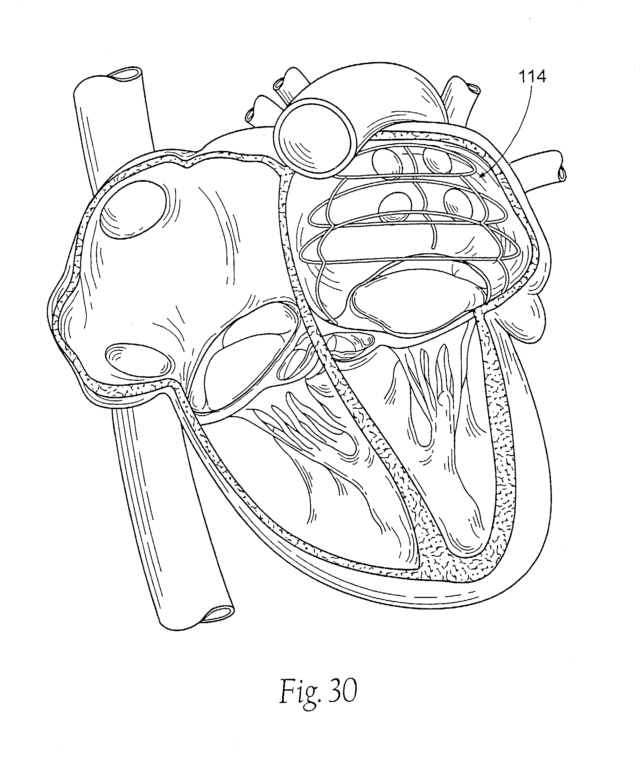

Fig. 30 shows another representative embodiment of a scaffold device 114. The scaffold device 114 is in the form of a hoop-and-strut wire cage that, when deployed, closely conforms to the interior of a patient's heart chamber, such as the patient's left atrium. The deployed scaffold device 114 may have a dome-shaped or tapered cylindrical configuration, with an upper hoop, a middle hoop and a lower hoop joined by longitudinal struts.

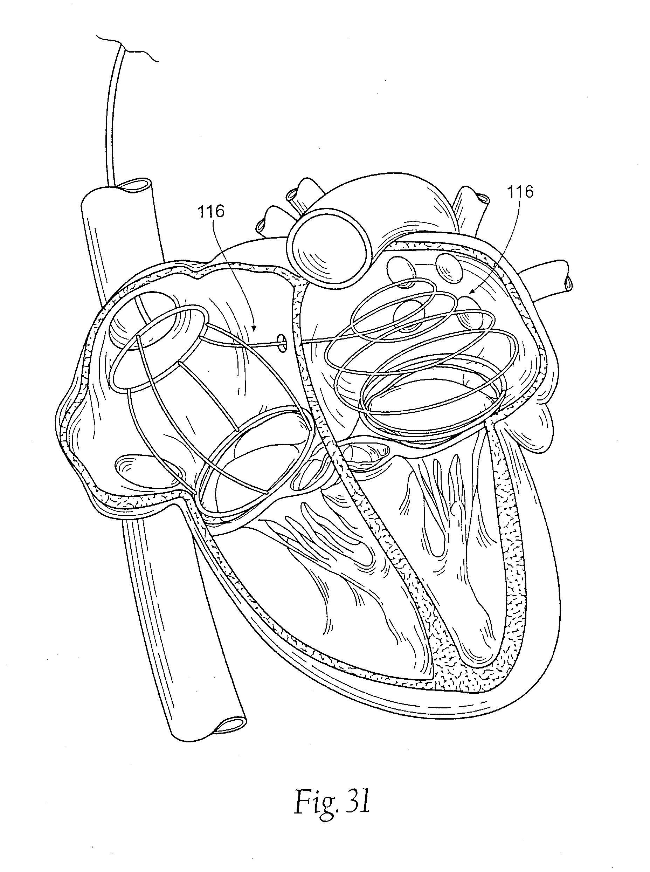

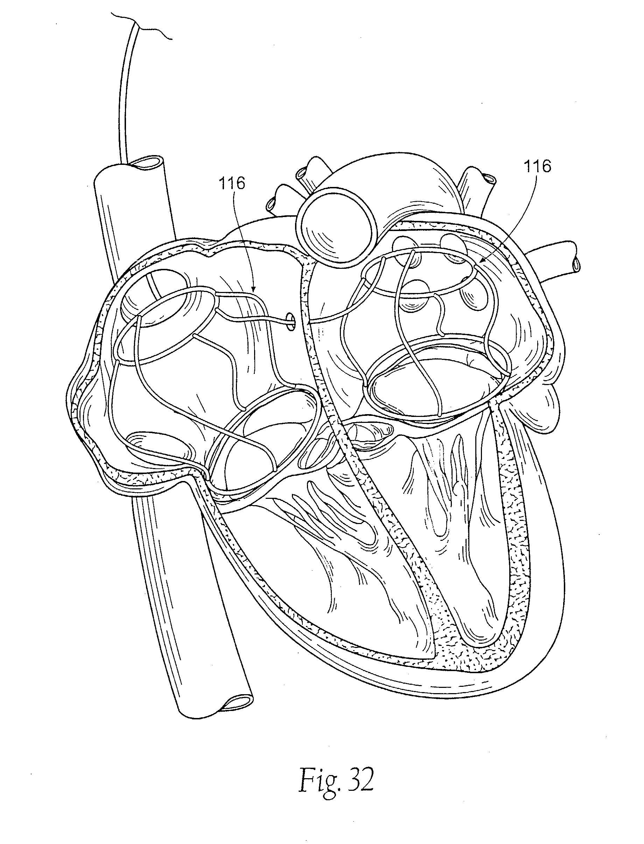

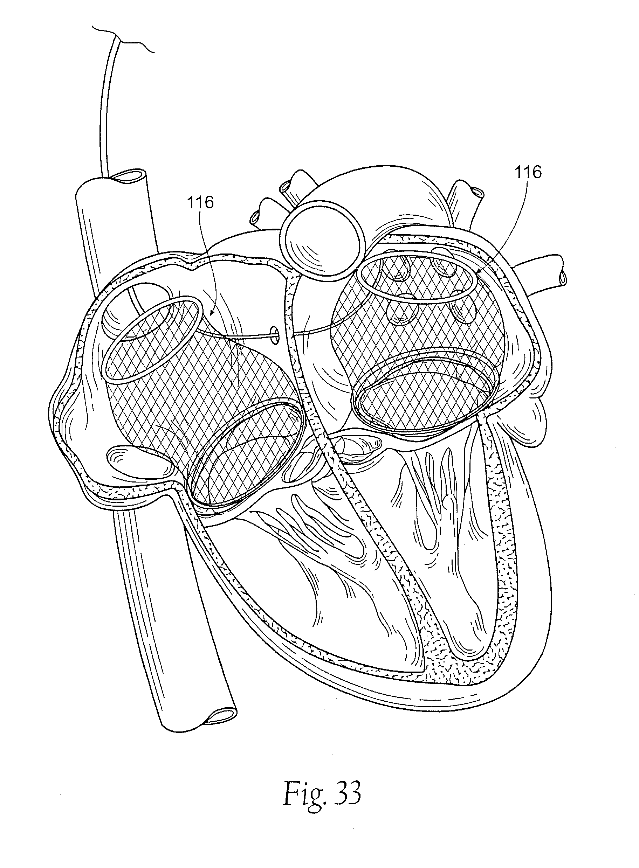

Figs. 31 to 33 show various representative embodiments of scaffold devices 116 deployed simultaneously in two heart chambers.

All of the above scaffold devices may possess ablation agent delivery sites 32 arranged in prescribed patterns suited to the therapeutic objective. All of the above scaffold devices may also possess catalytic energy sources for the purpose of enhancing the ablative effects of the ablation agents.

### F. Support for Other Functions

The many embodiments of devices described above support the creation of lesion patterns by ablation, including ablation in the region of the pulmonary veins, by use of an ablation agent. It should be appreciated that the device can support other therapeutic functions as well.

For example, a given tubule 38 can be free of delivery sites 32, and instead serve as a heat exchanger. In this arrangement, a heat removing fluid can be circulated within the tubule 38, giving rise to a conduction block in the adjacent tissue. Depending upon the time of exposure, the conduction block could be permanent or temporary. By the creation of temporary conduction blocks, a device of the type described can be used as a diagnostic tool, e.g., for determining the origin of ectopic pacemakers. Also, with longer exposures to adjacent tissues, the heat removal aspect of the device could result in tissue shrinkage, to tighten tissue within the heart, e.g., for promoting valve function, or to close off an atrial appendage, etc.

A given device could also support other therapeutic or diagnostic functions as well. Instead of carrying catalytic energy sources, a device could carry one or more defibrillating sources, and/or pacemaking sources, and/or electronic sensing devices. In this arrangement, a given device can serve therapeutic or diagnostic functions, such as heart function sensing, and/or ablation-pacer sensing, and/or electrical resistance measuring. It is believed that a sensor pacing array could comprise an array of electrodes located along a tubule 38 at intervals ranging from about 5mm to about 30 mm. Using a scaffold device to support a sensor pacing array allows pacing in a heart that is not in active atrial fibrillation at baseline, and then to repeat pacing after ablation to determine if the ablation is complete. If ablation is complete, pacing will not propagate to capture the heart. If pacing captures the heart, then ablation is incomplete.

### II. EPICARDIAL HEART TREATMENT DEVICE

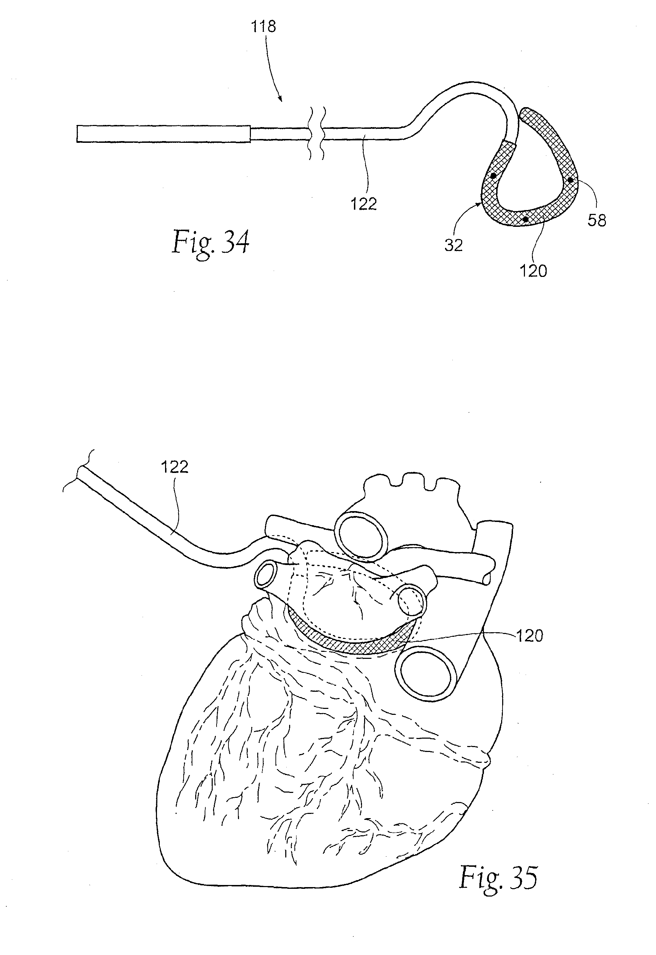

Figs. 34 and 35 show an epicardial heart treatment device 118. The epicardial heart treatment device 118 shares the functions and technical features that the endocardial heart treatment device 10 shown in Figs. 1A and 1B possesses, except that the epicardial heart treatment device 118 shown in Figs. 34 and 35 is sized and configured, in use, to be positioned on an epicardial surface of the heart surrounding the four pulmonary veins (see Fig. 35). The epicardial heart treatment device 118 is sized and configured so that, in use, it establishes a stable platform, or scaffold, that makes it possible deliver an ablation agent (in liquid, gel, or wafer form) around the periphery of all four pulmonary veins.

The epicardial heart treatment device 118 may be constructed in various ways. In the embodiment shown in Figs. 34 and 35, the device 118 includes a ring-like frame 120 with an attached shaft 122. The frame 120 can be made of biocompatible metallic or polymeric materials, or combinations thereof. The frame 120 is flexible and sized and configured to be placed into contact with an epicardial tissue surface using, e.g., a thoracoscopic surgical approach, or an open chest thoracotomy, or a sternotomy. These surgical approaches typically do not require cardiopulmonary bypass, but cardiopulmonary bypass may be used, if desired. The frame 120 allows it to be opened, flexed, and fitted, by use of grasping instruments guided by direct visualization or thoracoscopic means, about the pulmonary trunk into contact with epicardial tissue, such that the frame substantially encircles all four pulmonary veins (see Fig. 35).

The undersurface of the frame 120, i.e., the surface that makes contact with the epicardial heart surface, includes an array of sites 32 for the delivery of the ablation agent. The sites may comprise micro-ports (with or without a permeable membrane), micro-needles, or needle-less injection ports, as already described in the connection with the endocardial heart treatment device 10.

As also before described, the ablation agent may comprise a tissue fixative or drug agent in liquid or hydrogel form. The agent may be extruded from the delivery sites 32 directly onto epicardial tissue surrounding the pulmonary veins. Such agents could also be placed directly by hand without use of the treatment device.

The epicardial heart treatment device 118 may also carry catalytic energy sources 58, as previously described, to enhance or accelerate the delivery of the ablation agent.

In use, the delivery of the ablation agent by the device 118 forms a lesion pattern in epicardial tissue that substantially encircles the pulmonary veins. This results in epicardial surface isolation of the pulmonary veins.

It should be appreciated that the epicardial device 118 can be used to perform other therapeutic and/or diagnostic functions affecting epicardial tissue, e.g., to pace heart tissue or to analyze heart function for diagnostic purposes, e.g., to locate the origin of ectopic pacemakers or other regions where therapeutic intervention may be indicated.

In addition, other embodiments and uses of the invention will be apparent to those skilled in the art from consideration of the specification and practice of the invention disclosed herein. The specification and examples should be considered exemplary and merely descriptive of key technical features and principles, and are not meant to be limiting. The true scope and spirit of the invention are defined by the following claims. As will be easily understood by those of ordinary skill in the art, variations and modifications of each of the disclosed embodiments can be easily made within the scope of this invention as defined by the following claims.

## Claims

1. An endocardial treatment device comprising a scaffold structure sized and configured to rest within a left atrium between a septum wall and an opposite lateral wall, the scaffold structure supporting a first component sized and configured to rest adjacent to tissue on a posterior wall essentially surrounding a pulmonary vein region, the scaffold structure also supporting a second component configured to rest adjacent to tissue on the posterior wall and overlay a portion of a coronary sinus and a portion of a posterior mitral valve annulus within the left atrium.

2. A device according to claim 1, wherein at least one of the first and second components includes at least one therapeutic element.

3. A device according to claim 3, wherein the therapeutic element comprises a tissue ablating element.

4. A device according to claim 1, wherein at least one of the first and second components includes at least one diagnostic element.

5. A device according to claim 4, wherein the diagnostic element comprises a heart function sensing element.

6. A device according to claim 1, wherein at least one of the first and second components includes at least one energy delivery component.

7. A device according to claim 1, wherein at least one of the first and second components includes at least one ablation agent delivery site.

8. A device according to claim 7, wherein the ablation energy delivery site includes a micro-port.

9. A device according to claim 7, wherein the ablation energy delivery site includes a micro-needle.

10. A device according to claim 7, wherein the ablation energy delivery site includes a needle-less injection port.

11. A device according to claim 1, wherein at least one of the first and second components includes an array of ablation energy delivery sites.

12. A device according to claim 1, wherein at least one of the first and second components includes at least one catalytic energy source to affect intake of an ablation agent by tissue.

13. A device according to claim 12, wherein the catalytic energy source comprises at least one of an ultrasound source, and a radiofrequency microwave source, and an electrophoresis source, and a heat source.

14. A device according to claim 1, wherein at least one of the first and second components includes at least one ablation agent delivery site and at least one catalytic energy source to affect intake of ablation agent by tissue.

15. A device according to claim 1, wherein at least one of the first and second components includes at least one ablation agent delivery site, and further including at least one catalytic energy source to affect intake of ablation agent by tissue.

16. A device according to claim 15, wherein the catalytic energy source is carried by the scaffold structure.

17. A device according to claim 15, wherein the catalytic energy source is implanted.

18. A device according to claim 1, wherein the scaffold structure is free of a component that contacts tissue along an anterior wall of the left atrium.

19. A device according to claim 1, wherein the scaffold structure includes a distal strut that engages tissue at, within, or beneath the mitral valve annulus.

20. A device according to claim 1, wherein at least one of the first and second components includes at least one ablation agent delivery site, and further including a source of an ablation agent coupled to the ablation agent delivery site.

21. A device according to claim 20, wherein the ablation agent comprises a tissue fixative.

22. A device according to claim 21, wherein the tissue fixative includes alcohol, or ethanol, or DMSO, or acetone.

23. A device according to claim 20, wherein the ablation agent is in liquid form.

24. A device according to claim 20, wherein the ablation agent comprises a hydrogel formulation.

25. A device according to claim 1, wherein at least one of the first and second components includes at least one ablation agent delivery site located along a mural facing surface of the at least one component and not along a surface facing a blood volume.

26. A device according to claim 1, wherein the first component includes a superior element sized and configured to rest adjacent to tissue above the pulmonary vein region and an inferior element sized and configured to rest adjacent to tissue below the pulmonary vein region.

27. A device according to claim 26, wherein the second component extends from the inferior element.

28. A device according to claim 1, wherein the scaffold structure includes an elastic material.

29. A device according to claim 1, wherein the scaffold structure is sized and configured for delivery to the left atrium in a compressed condition within a catheter.

30. An endocardial treatment system comprising a scaffold structure as defined in claim 1, and an ablation element sized and configured to be deployed in the coronary sinus in a region overlaid by the second component.

31. A method of treating tissue in a left atrium comprising using the device as defined in claim 1.

32. A method according to claim 31, further including deploying an ablation element in the coronary sinus in a region overlaid by the second component.

33. An endocardial treatment device comprising a scaffold structure sized and configured to rest within a left atrium between a septum wall and an opposite lateral wall, the scaffold structure supporting a component sized and configured to rest adjacent to tissue on a posterior wall essentially surrounding a pulmonary vein region, the scaffold structure being free of a component that contacts tissue along an anterior wall of the left atrium.

34. A device according to claim 1, wherein the scaffold structure includes a distal strut that engages tissue at, within, or beneath the mitral valve annulus.

35. A device according to claim 33, wherein the component includes at least one therapeutic element.

36. A device according to claim 35, wherein the therapeutic element comprises a tissue ablating element.

37. A device according to claim 33, wherein the component includes at least one diagnostic element.

38. A device according to claim 37, wherein the diagnostic element comprises a heart function sensing element.

39. A device according to claim 33, wherein at least one of the first and second components includes at least one energy delivery component.

40. A device according to claim 33, wherein the scaffold structure includes a distal strut that engages tissue at, within, or beneath the mitral valve annulus.

41. A device according to claim 33, wherein the component includes at least one ablation agent delivery site.

42. A device according to claim 41, wherein the ablation agent comprises a tissue fixative.

43. A device according to claim 42, and further including at least one catalytic energy source to affect intake of ablation agent by tissue.

44. A device according to claim 43, wherein the catalytic energy source is supported by the scaffold structure.

45. A device according to claim 43, wherein the catalytic energy source is implanted.

46. A device according to claim 43, wherein the catalytic energy source comprises at least one of an ultrasound source, and a radiofrequency microwave source, and an electrophoresis source, and a heat source.

47. A method of treating tissue in a left atrium comprising using the device as defined in claim 33.

48. A heart treatment device comprising a scaffold structure sized and configured to rest adjacent to epicardial tissue essentially surrounding a pulmonary vein region, the scaffold structure supporting an ablation agent delivery site and at least one catalytic energy source to affect intake of ablation agent by tissue.

49. A method of treating epicardial tissue comprising using the device as defined in claim 48.

50. A heart treatment device comprising a scaffold structure sized and configured to rest adjacent to endocardial tissue essentially surrounding a pulmonary vein region, the scaffold structure supporting an ablation agent delivery site and at least one catalytic energy source to affect intake of ablation agent by tissue.

51. A method of treating endocardial tissue comprising using the device as defined in claim 50.

52. A heart treatment device comprising a scaffold structure sized and configured to rest adjacent to endocardial tissue, the scaffold structure supporting a therapeutic agent delivery site.

53. A method of treating endocardial tissue comprising using the device as defined in claim 52.
